# EUROPEAN PATENT SPECIFICATION

(11) **EP 4 374 825 B1**
(45) Date of publication and mention of the grant of the patent: **02.09.2026**
(21) Application number: 24170180.4
(22) Date of filing: 14.04.2020
(51) Int. Cl.: A61F 2/92, A61F 2/18, A61F 2/95

(54) **SINUS STENT AND SYSTEMS**
SINUSSTENT UND SYSTEME
STENT ET SYSTÈMES POUR SINUS

(30) Priority: 16.04.2019 US 201962834628 P
(43) Date of publication of application: 29.05.2024
(62) Document number(s) of the earlier application(s) in accordance with Art. 76 EPC: 20720939.6 / 3 955 860
(73) Proprietor: Stryker European Operations Limited, Carrigtwohill, Co. Cork T45 HX08 (IE)
(72) Inventor: JACOBS, Johannes Jacobus, Limerick, V94 VE09 (IE); CUSHEN, Patrick, Cork Shanagarry, P25AX88 (IE)
(74) Representative: Röthinger, Rainer

(56) References cited:
- US-A1- 2007 207 186
- US-A1- 2010 106 255

## Description

### PRIORITY CLAIM

This application claims priority to and all the benefits of United States Provisional Patent Application No. 62/834,628, filed April 16, 2019.

### BACKGROUND

Chronic rhinosinusitus (CRS) represents one of the most common healthcare problems in the United States. When sinusitis occurs, the mucociliary clearance mechanism works less efficiently, preventing the proper flow of mucus. The mucous membranes of the sinuses become engorged, resulting in ostia closure. Poor ventilation and accumulation of mucus then produce the conditions required for bacterial infection.

Currently, the medical management of CRS encompasses a broad spectrum of therapeutic modalities including oral antibiotics and corticosteroids, saline irrigations, immunomodulators, etc., but these methods are typically inefficient. For example, the amount of active agent reaching the target sinunasal tissue from nasal sprays is very low, as a large proportion has been found to be expelled through the gastrointestinal tract.

Functional endoscopic sinus surgery (FESS) is now a well-established, minimally invasive treatment option for patients with sinunasal disease. Further, it is known that long-term stenting for a period of several months significantly after FESS reduces the rate of restenosis. Document US 2010/0106255 A1 relates to a device for stenting of the frontal sinus, and more particularly to a self-expanding stent for use in keeping the frontal sinus pathway open and an insertion tool having the appropriate angulation and malleability to facilitate ease of insertion of the stent into the frontal sinus. Further, document US 2007/0207186 A1 relates to improved materials and configurations for use in the manufacturing of medical devices such as expandable intraluminal stents, grafts, stent-grafts, surgical fabrics, suppositories, transdermal patches, or oral medications that optionally deliver active ingredients within a living body. However, there is a lack of adequate commercially available products that promote frontal sinus patency and mucosal recovery. Therefore, there is a need in the art for an improved stent and delivery system that allows for administration of active agents directly into the mucosal wall in a manner to facilitate drainage from the frontal sinus, and to help maintain patency between the frontal sinus cavity and the nasal cavity following FESS.

### SUMMARY

A first aspect is directed to a stent positionable within a frontal sinus cavity. The stent includes a flexible foam layer having a porosity of greater than 80%. The flexible foam layer comprising a polyurethane including amorphous segments, and crystalline segments formed via hydrogen bonding. An active agent is within the flexible foam layer. The stent further includes a flexible film layer arranged in a stacked configuration and including a polymer which is hydrogen bonded to the crystalline segments of the flexible foam layer at a bond interface. The flexible foam layer and the flexible film layer are configured to be furled to assume a substantially cylindrical profile prior to insertion within the frontal sinus cavity such that the flexible foam layer at least partially defines an outer annular aspect of the stent and the flexible film layer at least partially defines an inner annular aspect of the stent. The flexible film layer has a resilience sufficient to unfurl the stent and urge the outer annular aspect of the flexible foam layer into direct contact with mucosa of the frontal sinus cavity when positioned in an unrestricted state in the frontal sinus cavity.

In some implementations, removal of the flexible film layer from the flexible foam layer results in cohesive failure of the foam layer at the bond interface. The bond interface may be free of adhesive and the flexible film layer and the flexible foam layer are bonded directly to one another via hydrogen bonding. The flexible film layer and the flexible foam layer may be substantially free of covalent bonds therebetween.

In some implementations, the crystalline segments of the flexible foam layer comprise the reaction product of 1,4 butanediol and 1,4-diisocyanatobutane. Molecules within the polyurethane foam layer may be arranged so that the crystalline segments and the amorphous segments stack in an alternating configuration to provide a 3-dimentional porous structure which is strengthened via hydrogen bonding between the stacked crystalline segments.

In some implementations, the stent includes a first body portion and a second body portion coupled at a junction extending partially between opposing sides of the stent such that the first and second body portions are configured to be independently furled and/or unfurled other than at the junction. Each of the first and second body portions may include the flexible foam layer and the flexible film layer. The first and second body portions may be separated at a boundary defined between the opposing sides and including the junction. The stent may further include cutouts extending inwardly from the opposing sides with the cutouts including a first aspect associated with the first body portion and a second aspect associated with the second body portion. Each of the first and second aspects of the cutout may be angled relative to the boundary. An angle of the first aspect of the cutouts relative to the boundary may be within a range of approximately 25 degrees and 75 degrees. An angle of the second aspect of the cutouts relative to the boundary may be within a range of approximately 25 degrees and 75 degrees. The first and second aspects may be continuous with one another and form the cutouts that are substantially parabolic in shape. The flexible foam layer may have a first thickness defined between a first outer face and the interface, and the flexible film layer may have a second thickness defined between a second outer face and opposite the first outer face and the bond interface. The first thickness may be greater than the second thickness. The opposing sides may be first opposing sides with the stent further including second opposing sides extending between the first opposing sides. The second opposing sides may be arcuate in shape and intersecting with the first opposing sides at rounded corners.

In some implementations, the flexible film layer includes polysiloxane. The flexible film layer may include polyurethane. The flexible film layer may include a bioresorbable polymer comprising silanol groups and/or urethane groups. The flexible foam layer may be bioresorbable.

In some implementations, the active agent includes a molecule including at least one hydrogen atom which is bound to a nitrogen, oxygen, or fluorine atom. The active agent may be selected from corticosteroids, hemostatic agents, and combinations thereof.

A second aspect of the disclosure is directed to a method of deploying the stent according to the first aspect of the disclosure, and optionally, any of its corresponding implementations.

A third aspect of the disclosure is directed to a disposable cartridge assembly for deploying the stent according to the first aspect of the disclosure, and optionally, any of its corresponding implementations.

In some implementations, the cartridge assembly includes a tubular sheath comprising at least one sidewall defining a proximal end, a distal end opposite the proximal end, and a lumen extending between the proximal and distal ends and defining a sheath volume sized to receive substantially an entirety of the furled stent between the proximal and distal ends. A cap portion may be coupled to the proximal end of the tubular sheath. The cap portion may include a cap body, a lip, and coupling features. The cap body may define an aperture in communication with the lumen of the tubular sheath and sized to receive a plunger element of the applicator device. The lip may extend outwardly from the cap body to define a surface configured to abut a distal end of the applicator device when the disposable cartridge is coupled with the applicator device. The coupling features may be disposed on the cap body with the coupling features configured to removably engage complimentary coupling features of the applicator device. The tubular sheath may be flexible and sized to be inserted into the frontal sinus cavity. The lip may be annularly disposed about the cap body having a cylindrical profile. The aperture of the cap body may be coaxial with the lumen of the tubular sheath. Respective diameters of the lumen of the tubular sheath and the aperture of the cap body may be substantially equal. The coupling features may include deflectable fingers circumferentially spaced about the cap body.

A fourth aspect of the disclosure is directed to a method of operating the cartridge assembly according to the third aspect of the disclosure to deploy the stent according to the first aspect of the disclosure, and optionally, any of its corresponding implementations.

A fifth aspect of the disclosure is directed to a system for stenting a frontal sinus cavity. An applicator device includes a housing, an actuator, and a drive member coupled to the actuator. The housing defines a bore and is adapted to be grasped with a single hand of a user. The drive member is configured to be moved within the bore in response to one or both of the housing and the actuator receiving an input from the user. A disposable cartridge is removably coupled to the housing and includes a tubular sheath defining a lumen, and a cap portion coupled to the tubular sheath and removably coupled to the housing of the applicator device. The system includes the stent according to the first aspect of the disclosure, and optionally, any of its corresponding implementations. The stent is configured to be furled and inserted within the tubular sheath. The flexible film layer having a resilience sufficient to unfurl the stent when positioned in an unrestricted state in the frontal sinus cavity.

In some implementations, the system includes the cartridge assembly according to the third aspect of the disclosure, and optionally, any of its corresponding implementations.

In some implementations, an inner annular aspect of the furled stent defines an interior passageway. The drive member may include a distal end opposite a proximal end coupled to the actuator. A mandrel may extend from the distal end and through the interior passageway of the stent when the stent is disposed within the flexible sheath. The cap portion may define an aperture in communication with the lumen of the tubular sheath. The mandrel of the drive member may extend through the aperture of the cap portion when the cap portion is coupled to the housing. The drive member further may include a plunger element with the mandrel extending from the plunger element. The aperture of the cap portion is sized to receive the plunger element of the applicator device when the cap portion is coupled to the housing. The aperture may be sized to movably receive the drive member of the applicator device such that the drive member extends through the aperture and the lumen of the tubular sheath when the stent is deployed from the disposable cartridge. The housing and the cap portion may further include complementary coupling features configured to removably couple the disposable cartridge with the applicator device. At least a portion of the housing may be flexible and at least a portion of the drive member may be malleable and configured to receive another input from the user imparting a desired curve of the disposable cartridge, and to maintain the desired curve of the disposable cartridge relative to the housing upon release of the another input. The actuator may include a push rod movably disposed within the bore of the housing.

A sixth aspect of the disclosure is directed to a method of operating the system according to the fifth aspect of the disclosure, and optionally, any of its corresponding implementations.

A seventh aspect is directed to a stent positionable within a frontal sinus cavity. The stent includes a flexible foam layer, and a flexible film layer arranged in a stacked configuration. The flexible foam layer and the flexible film layer are configured to be furled prior to insertion within the frontal sinus cavity. The flexible film layer has a resilience sufficient to unfurl the stent and urge the flexible foam layer into direct contact with mucosa of the frontal sinus cavity when positioned in an unrestricted state in the frontal sinus cavity.

In some implementations, the stent of the seventh aspect may include any of the corresponding implementations of the stent according to the first aspect of the disclosure. The stent may be deployed with the disposable cartridge assembly according to the third aspect of the disclosure, and optionally, any of its corresponding implementations, and operable with the system according to the fifth aspect of the disclosure, and optionally, any of its corresponding implementations.

### BRIEF DESCRIPTION OF THE DRAWINGS

Advantages of the disclosure will be readily appreciated as the same becomes better understood by reference to the following detailed description when considered in connection with the accompanying drawings.
Figure 1 is a perspective view of a system for deploying a stent into a sinus cavity.
Figure 2 is an exploded view of the system of Figure 1.
Figure 3 is a sectional view of the system of Figure 1 taken along section lines 3-3.
Figure 4A is an elevation view of a stent in an unrestricted state.
Figure 4B is an elevation view of another stent in an unrestricted state.
Figure 4C is an elevation view of another stent in an unrestricted state.
Figure 4D is an elevation view of another stent in an unrestricted state.
Figure 5 is a plan view of a stent in an unrestricted state and including a flexible film layer and a flexible foam layer arranged in a stacked configuration.
Figure 6 is a plan view of a stent including a first body portion unfurled and a second body portion furled.
Figure 7 is an elevation view of a stent including the first body portion unfurled and the second body portion furled.
Figure 8 is an elevation view of another stent with the first body portion unfurled and the second body portion furled.
Figure 9 is a perspective view of a stent in an unfurled configuration prior to insertion into a disposable cartridge.
Figure 10 is a perspective view of the stent in a furled configuration prior to insertion into the disposable cartridge.
Figure 11 is a perspective view of the stent disposed within the disposable cartridge.
Figure 12 is a perspective view of the stent disposed within the disposable cartridge prior to directing a mandrel of an applicator device through an interior passageway defined by the stent.
Figure 13 is a perspective view of the stent disposed within the disposable cartridge prior to coupling the disposable cartridge with the applicator device.
Figure 14 is a perspective view of the stent disposed within the disposable cartridge coupled with the applicator device.
Figure 15 is a perspective view of the system prior to movement of a housing relative a plunger of the applicator device.
Figure 16 is a perspective view of the system after movement of the housing relative to the plunger to deploy the stent beyond the disposable cartridge.
Figure 17 is an illustration of a portion of a human head identifying certain structures and regions of the frontal sinus.

### DETAILED DESCRIPTION

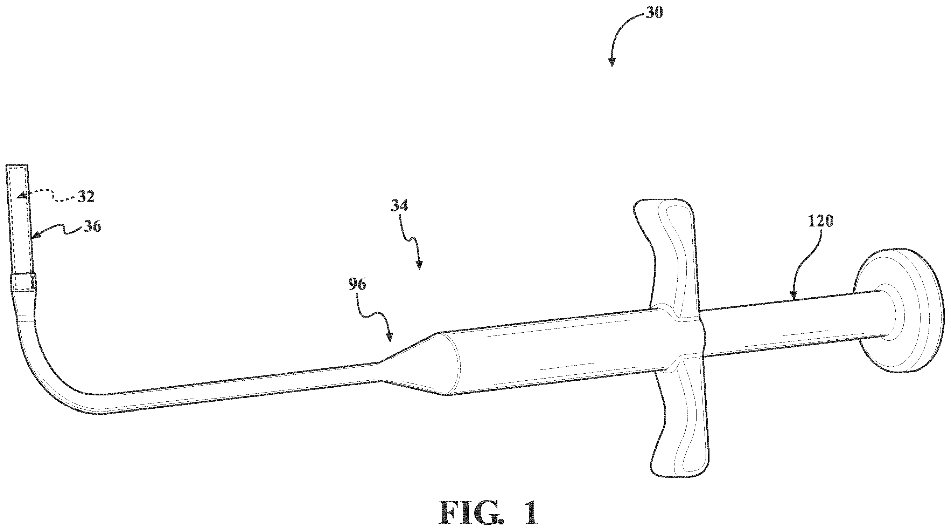

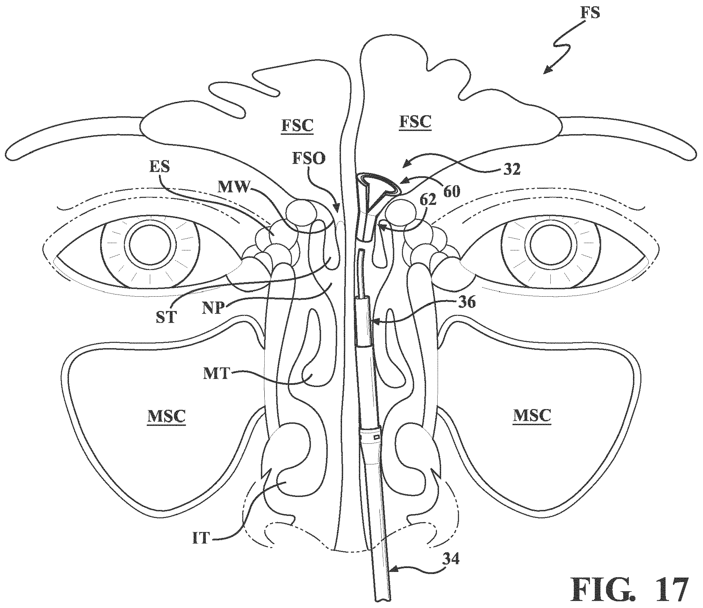

Figures 1-3 show a system 30 for deploying a stent 32 into a frontal sinus (FS) of a patient (see Figure 17). The system 30 addresses challenges of difficult visualization and complex anatomy often associated with frontal sinus surgery. The system 30 may be particularly well suited for functional endoscopic sinus surgery (FESS), a minimally invasive surgical technique in which sinus air cells and sinus ostia are opened under direct visualization. In one example, the stent 32 is deployed within the frontal sinus following FESS to reduce, minimize, and/or eliminate restenosis of the frontal sinus opening (FSO) (*i.e.,* ostium), one of the primary postsurgical indicators of long term outcome associated with chronic rhinosinusitus (CRS). The system 30 also may be utilized to treat inflammation, muscosal edema, polyposis, and adhesives, among other disorders, thereby decreasing recurrent symptomatology and/or the need for further surgical intervention. Moreover, in manners to be further described, the system 30 may facilitate improved delivery for topical administration of therapies (*e.g.,* antibiotics, corticosteroids, immunomodulators, etc.) to the mucosal wall in which a greater portion of the active agent in the therapies reach the target tissue.

The system 30 includes the stent 32, an applicator device 34, and a cartridge 36 with each to be described in turn. The applicator device 34, in a broadest sense, is configured to receive an input from a user and deploy the stent 32 from the cartridge 36.

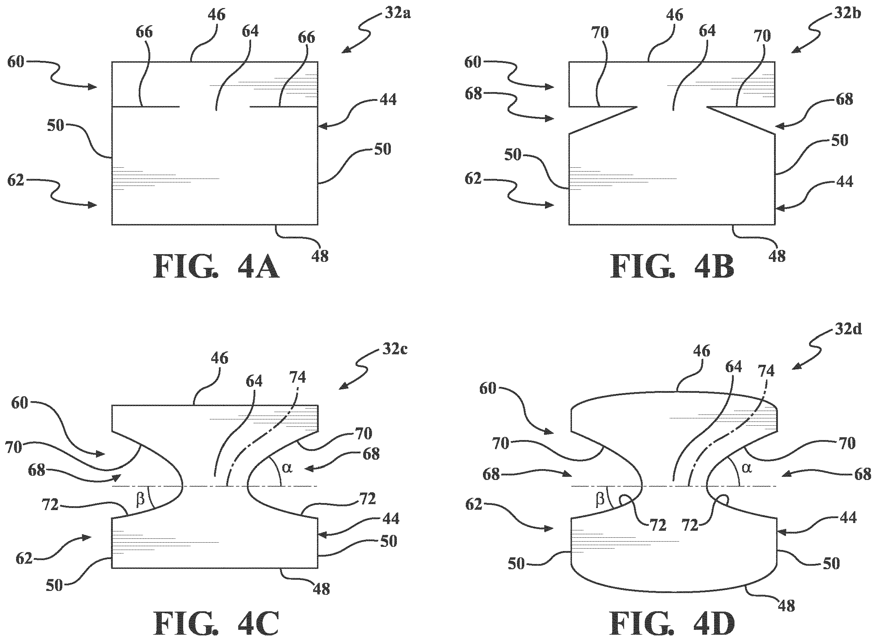

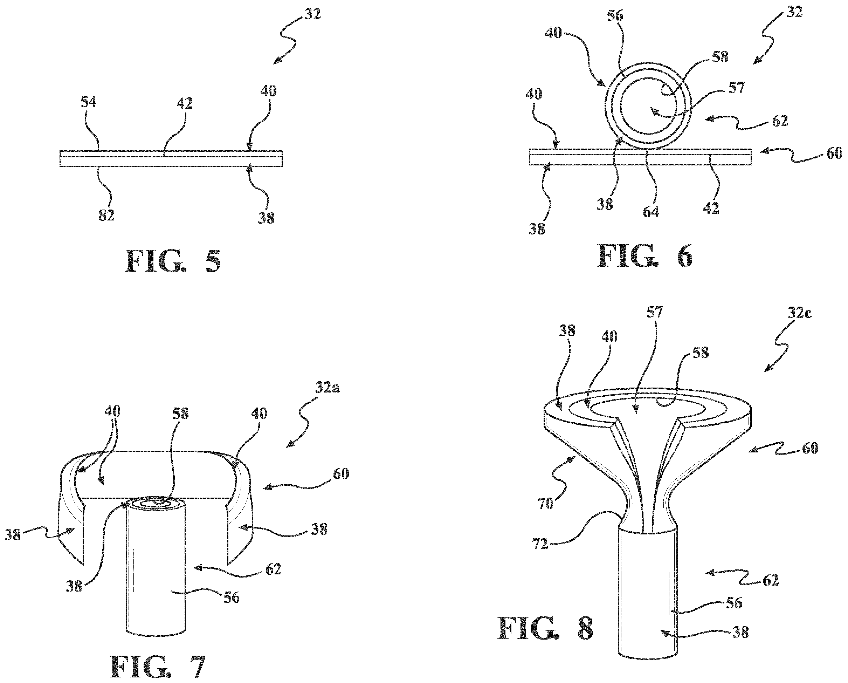

With further reference to Figures 4A-8, the stent 32 includes a flexible foam layer 38, and a flexible film layer 40 coupled to the flexible foam layer 38, for example bonded to the flexible foam layer 38 at a bond interface 42. The flexible foam layer 38 and the flexible film layer 40 may be arranged in a stacked configuration with the flexible foam and film layers 38, 40 having common profiles, shapes, and/or dimensions. For example, Figure 4A shows one variant of the stent 32*a* that is rectangular in plan with an outer perimeter 44 of the stent 32 defined by upper 46, lower 48, and opposing lateral sides 50. Each of the flexible foam and film layers 38, 40 may share or define a portion of the upper 46, lower 48, and opposing lateral sides 50. In other words, the upper 46, lower 48, and opposing lateral sides 50 may be at least partially defined by a thickness (see, *e.g.,* Figure 5) of each of the flexible film layer 38 and the flexible foam layer 40.

The thicknesses of the flexible foam and film layers 38, 40 may be the same or different. In one example, the flexible foam layer 38 has a first thickness defined between a first outer face 52 and the bond interface 42, and the flexible film layer 40 has a second thickness defined between the bond interface 42 and a second outer surface 54 opposite the first outer surface 52. Figure 5 shows the thickness of the flexile foam layer 38 being greater than the thickness of the flexible film layer 40. It is contemplated that additional layers may be included in the stent 32.

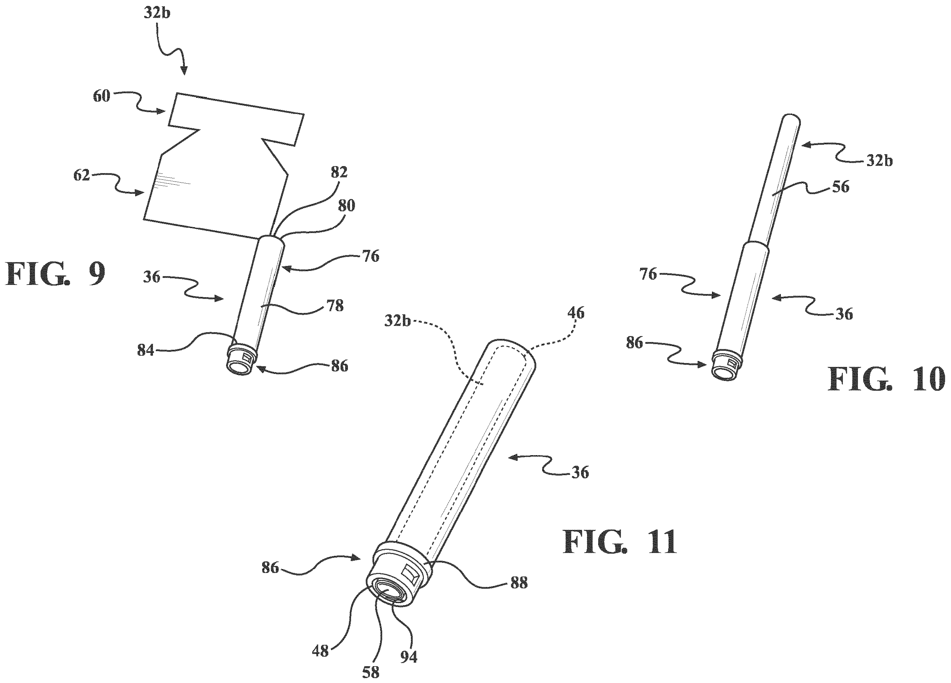

The stent 32 is configured to be furled to assume a substantially cylindrical profile for insertion through the nasal passage (NP) and through the frontal sinus opening to within the frontal sinus cavity (FSC) (see Figures 10 and 17), and the flexible film layer 40 has a resilience sufficient to unfurl the stent 32 once positioned and unrestricted within the frontal sinus cavity. Stated differently, the stent 32 may be sufficiently thin to be rolled onto itself while also being able to recoil itself subsequent to insertion within the frontal sinus cavity. When partially or fully furled, an outer annular aspect 56 of the stent 32 has a dimension small enough to be inserted through, for example, the nasal passageway, the sinus cavity opening, the frontal sinus cavity, and the like. The unfurling or recoiling facilitates patency of the frontal sinus cavity and promotes improved drainage. Moreover, this is accomplished with the resilience of the material(s) forming the flexible film layer 40 as opposed to known devices requiring discrete stiffening elements or the like. Such known devices may be associated with areas of focal pressure on sensitive anatomy of the frontal sinus cavity and/or areas of varying patency of an interior passageway defined by the device. Among other advantages to be explained, the stent 32 of the disclosure achieves more even unfurling for improved patient comfort and generally uniform patency of an interior passageway 57 (see Figure 6) defined by the furled or partially unfurled stent 32 within the frontal sinus. An inner annular aspect 58 opposite the outer annular aspect 56 may define the interior passageway 57 of the substantially cylindrical profile of the stent 32 in when furled.

Returning to Figures 4A-4D, the stent 32 may include a first body portion 60 and a second body portion 62. Each of the first and second body portions 60, 62 may each include the flexible foam and film layers 38, 40. The first and second body portions 60, 62 are coupled to one another at a junction 64 such that the stent 32 may be of unitary construction. The stent 32*a* of Figure 4A shows the junction 64 generally defined between slits 66 extending inwardly from the opposing sides 50 of the stent 32. The first body portion 60 on one side of the slits 66 may have a length greater than a length of the second body portion 62 on the other side of the slits 66. The first and second body portions 60, 62 are configured to independently furled and unfurled relative to one another except for at the junction 64. With the first and second body portions 60, 62 configured to independently furled and unfurled relative to one another, the first body portion 60 may facilitate retaining the stent 32 within the frontal sinus cavity. In manners to be further explained, the stent 32 is positionable through the frontal sinus opening with the first body portion 60 within the frontal sinus cavity and the second body portion 62 within the nasal passage (NP) in communication with the frontal sinus cavity opposite the frontal sinus opening. Based on the relative sizes of the frontal sinus cavity and the nasal passage, the first body portion 60 unfurls to a greater extent than the second body portion 62, and more particularly to an extent to create interference with the anatomy defining the frontal sinus opening to retain the stent 32 within the frontal sinus (FS). The second body portion 62 unfurls to a lesser extent than the first body portion 60 to be seated or urged against the mucosal wall (MW) of the frontal sinus opening, thereby maintaining sinus patency as well as delivering an active agent within the flexible foam layer to the mucosal wall (see Figure 17).

In another variant of the stent 32*b* shown in Figure 4B, cutouts 68 extending inwardly from the opposing sides 50 of the stent 32 to define the junction 64. The cutouts 68 may be defined between an upper aspect 70 associated with the first body portion 60, and a lower aspect 72 associated with the second body portion 62. The slits 66 or cutouts 68 may be prefabricated or completed by the physician near or at the time of placement. In one example, the slits 66 or cutouts 68 may be cut using conventional scissors.

With the stent 32 deployed within the frontal sinus and with the first body portion 60 creating interference with the anatomy defining the frontal sinus opening, eventually the stent 32 may need to be removed from the frontal sinus. The interference of the first body portion 60 with the anatomy needs to be overcome to remove the stent 32. This may include applying a pulling force to the stent 32, for example with a surgical instrument, upon which the first body portion 60 collapses (*e.g.,* partially furls) onto itself as the stent 32 is removed through the frontal sinus opening and the nasal passageway. The first body portions 60 of Figures 4A and 4B, for example the upper aspect 70 is oriented generally perpendicular to the direction of removal of the stent 32, which may require unnecessary pulling force and/or irritate the sensitive anatomy of the frontal sinus. Referring now to Figures 4C and 4D, variants of the stents 32*c*, 32*d* are shown with cutouts 68 shaped to ease the removal of the stent 32. In particular, the upper and/lower aspects 70, 72 are angled relative to a boundary 74 defining the junction 64. Figures 4C and 4D show the boundary 74 extending between the opposing lateral sides 50 of the stent 32. The upper aspect 70 is oriented upwardly at an angle, α, relative to the boundary 74. The angle α may be within a range of between approximately 25 to 75 degrees, and more particularly within a range of between approximately 40 to 60 degrees. The lower aspect 72 is oriented downwardly at an angle, β, relative to the boundary 74. The angle α may be within a range of between approximately 25 to 75 degrees, and more particularly within a range of between approximately 40 to 60 degrees. Further, an intersection of the upper and lower aspects 70, 72 may be contoured to remove any sharp edges. For example, the upper and lower aspects 70, 72 may be considered continuous with one another to form the cutouts 68 that are substantially parabolic in shape, as shown in Figures 4C and 4D. Likewise, Figure 4D shows the upper side 46 and the lower side 48 being arcuate in shape between the opposing lateral sides 50 and intersecting the opposing lateral sides 50 at rounded corners. The angularity of the upper aspect 70 facilitates the first body portion 60 formed from the flexible foam and film layers 38, 40 to more easily fold and collapse onto itself. Moreover, with the upper aspect 70 angled away from the direction of removal, forces from the upper aspect 70 contacting the sensitive nasal anatomy is appreciably reduced. Still further, the upper and lower aspects 70, 72 may facilitate ease with loading the stent 32 into the cartridge 36.

Referring now to Figures 7 and 8, variants of the stent 32*a*, 32*c* are shown in position approximating a deployed or unrestricted state within the frontal sinus. The flexible film layer 40 of the first body portion 60 unfurls the stent 32 against anatomy. The stent 32c of Figure 8 shows the first body portion 60 tapering based on the upper aspect 70. Further, the flexible film layer 40 of the second body portion 62 urges the outer annular aspect 56 of the flexible foam layer 38 into direct contact with the mucosal wall of the frontal sinus cavity. The inner annular aspect 58 of the flexible film layer 38 of the second body portion 62 defines the interior passageway 57 for drainage, respiration, and the like.

With the stent 32 including the flexible foam layer 38 bonded to the flexible film layer 40 in the stacked configuration, advantageous material properties associated with each of the layers 38, 40 may be fully realized. The bonding may be facilitated with the flexible foam layer 38 including a polyurethane including amorphous segments and crystalline segments, as to be described in greater detail. The properties of the flexible foam layer 38, in many examples, configured to swell (or not swell) upon exposure to moisture within the frontal sinus. To this end, the flexible foam layer 38 may include a phase-separated polymer. Further, it is possible to achieve good compressibility in which the phase-separated polymer of the flexible foam layer 38 retains its structure (in particular its compression strength) when having absorbed or being saturated with a liquid, such as blood or mucous. The mechanical, structural and chemical properties of the foamed phase-separated polymer are mainly determined by the composition (structure) of polymer used. To this end, selection of the reactants used to form the phase-separated polymer provide a way to control and adjust the mechanical, structural and chemical properties of the phase-separated polymer.

The term "phase-separated polymer" as used herein, refers to a polymer comprising soft (amorphous) segments, as well as hard (crystalline) segments, the hard segment having a phase transition temperature of at least mammalian body temperatures (which is generally 37°C for humans) and the phase-separated morphology being manifest when the foam prepared from such a polymer is applied in the human or animal body for a sufficient period of time. In addition, the polymer placed under temperature conditions comparable to the human or animal body exhibits the phase-separated morphology. A phase-separated polymer is characterized by the presence of at least two immiscible or partly miscible phases with a different morphology at normal environmental conditions. Within one material, a rubber phase and a crystalline phase (at a temperature above the glass transition temperature of the amorphous phase and below the melting temperature of the crystalline phase) may be present or a glassy and a crystalline phase (at a temperature below the glass transition temperature of the amorphous phase). Also at least two amorphous phases may be present at a temperature between the two phase transitions, *e.g.,* one glassy and one rubbery phase. At a temperature above the highest phase transition, which is either a melting or glass transition temperature, the liquid and rubbery or the two rubbery phases, respectively, may form a phase mixed morphology or they may still be immiscible. Immiscible liquid and/or rubbery phases usually results in a polymer with a phase-separated morphology without the initial desired mechanical properties at normal environmental conditions.

In some examples, the phase-separated polymer has a porosity of greater than 50, 60, 70, or 80%. Alternatively, the phase-separated polymer has a porosity from 50 to 99%, from 50 to 96%, from 60 to 96%, from 70 to 96%, from 80 to 93%, from 80 to 90%, from 80 to 87%, from 80 to 84%, from 83 to 99%, from 85 to 99%, from 89 to 99%, from 92 to 99%, from 95 to 99%, from 83 to 96%, from 86 to 93%, from 92-98%, from 95-98%, or 90%.

In some examples, the phase-separated polymer has a foam density of 0.01 to 1.0 g/cm³. Alternatively, the foam density may be from 0.01 to 0.5, 0.01 to 0.3, 0.01 to 0.1, 0.01 to 0.09, 0.01 to 0.08, 0.01 to 0.07, 0.01 to 0.06, 0.01 to 0.05, 0.01 to 0.04, 0.01 to 0.03, 0.02 to 0.08, 0.04 to 0.08, 0.05 to 0.08, 0.06 to 0.08, 0.02 to 0.08, or 0.03-0.07 g/cm³. In certain examples, the phase-separated polymer has a porosity of 85-99% and a foam density of 0.03-0.07 g/cm³. It is to be appreciated that the term "foam density" as used throughout this disclosure refers to the density of foam, calculated as the phase-separated polymer mass per volume unit of particular foam portion. Accordingly, if the particular foamed portion includes an active agent, the mass of the active agent present in the particular foamed portion is disregarded when calculating the foam density.

The phase-separated polymer may be selected from the group consisting of polyesters, polyethers, polyhydroxyacids, polylactones, polyetheresters, polycarbonates, polydioxanes, polyanhydrides, polyurethanes, polyester(ether)urethanes, polyurethane urea, polyamides, polyesteramides, poly-orthoesters, polyaminoacids, polyphosphonates, polyphosphazenes and combinations thereof. Such polymers are described in International Publication No. 99/64491.

As is described above, the phase-separated polymer includes soft (amorphous) segments, as well as hard (crystalline) segments. The term "amorphous" as used herein, refers to segments present in the phase-separated polymer with at least one glass transition temperature below the temperature of the cavities of the human or animal body into which the foam is packed, and may also refer to a combination of an amorphous and crystalline segment which is completely amorphous when packed in the human or animal body. For example, PEG in a pre-polymer may be crystalline in pure form, but may be amorphous when included in the R segment of a polyurethane of the formula (I). Longer PEG segments may also be partly crystalline when included in the R segment of a polyurethane of the formula (I), but will become amorphous ("dissolves") when placed in contact with water. Therefore such longer PEG segments are part of the soft segment of the phase-separated polymer of the formulas (I), whereas the hard segment should remain crystalline in nature to provide sufficient support for a particular foamed portion in the wet and packed state for a certain period of time.

The term "crystalline" as used herein, refers to segments, present in the phase-separated polymer, that are crystalline when packed in the human or animal body, *i.e.,* a melting temperature above the temperature of the human or animal body into which the closure device is inserted.

A "hydrophilic segment" as used herein, refers to a segment comprising at least one, preferably at least two, more preferably at least three hydrophilic groups such as may be provided for instance by C-O-C, or ether, linkages. A polyether segment may thus provide a hydrophilic segment. A hydrophilic segment may also be provided by polypeptide, poly(vinyl alcohol), polyvinylpyrrolidone) or poly(hydroxyethylmethacrylate). A hydrophilic segment is preferably derived from polyalkyleneglycol, such as polyethyleneglycol, polypropyleneglycol, or polybutyleneglycol. The preferred hydrophilic segment is a polyethyleneglycol (PEG) segment.

The term "segment" as used herein, refers to a polymeric structure of any length. In the art of polymer technology, a long polymeric structure is often referred to as a block, whereas a short polymeric structure is often referred to as a segment. Both these conventional meanings are understood to be included in the term "segment" as used herein.

In one particular example of the present application, the phase-separated polymer is of the formula:

-[R-Q₁[-R'-Z₁-[R"-Z₂-R'-Z₃]ₚ-R"-Z₄]_{q}-R'-Q₂]n- (I)

wherein R is selected from one or more aliphatic polyesters, polyetheresters, polyethers, polyanhydrides and/or polycarbonates, and optionally at least one R includes a hydrophilic segment, R' and R" are independently C₂-C₈ alkylene, optionally substituted with C1-C₁₀ alkyl or C1-C10 alkyl groups substituted with halides or protected S, N, P or O moieties and/or comprising S, N, P or O in the alkylene chain, Z₁-Z₄ are independently amide, urea or urethane, Q₁ and Q₂ are independently urea, urethane, amide, carbonate, ester or anhydride, n is an integer from 5-500, p and q are independent 0 or 1, provided that when q is 0, R is at least one amorphous aliphatic polyester, polyether, polyanhydride and/or polycarbonate segment with optionally at least one crystalline polyether, polyester, polyetherester or polyanhydride segment. The simplest form of the phase-separated polymer, as represented by formula (I), is of the formula: -R- Q₁-R'-Q₂-, *i.e.,* when q=0.

The amorphous segment is included in the -R- part of the polymer according to formula (I). In case q=l, the Q₁[-R'-Z₁-[R"-Z₂-R'-Z₃]ₚ-R"-Z₄]q-R'-Q₂ part of the polymer according to formula (I) represents the crystalline segment. In this particular example, the amorphous and crystalline segments are alternating, thus providing the hard segment with a uniform block-length.

As described above, R may represent a mixture of two or more different types of aliphatic polyesters, polyetheresters, polyethers, polyanhydrides and/or polycarbonates, which mixture includes both amorphous and crystalline types, so that both are included in a particular foamed portion. In the case that a mixture of amorphous and crystalline types of R segments are provided in a polymer according to the formula (I), optionally at least one hydrophilic segment is provided in at least one amorphous R segment. R may in particular be derived from the cyclic monomers lactide (L, D or LD), glycolide, ε-caprolactone, δ-valerolactone, trimethylenecarbonate, tetramethylenecarbonate, I,5-dioxepane-2-one, para-dioxanone, and combinations thereof and optionally polyethyleneglycol, polypropyleneglycol, polybutyleneglycol and combinations thereof. In certain examples, R is an amorphous polyester derived from exclusively lactide and ε-caprolactone, with a molecular weight between 1000 and 4000. In one example, R is about 25 wt.% lactide, about 25 wt.% ε- caprolactone and about 50 wt.% of polyethyleneglycol.

In a phase-separated polymer according to the formula (I), Q1 and Q2 may be selected from amide, urea, urethane ester, carbonate or anhydride groups, whereas Z1 through Z4 should be chosen from amide, urea or urethane groups so that at least 4 hydrogen bond forming groups are present in a row in the crystalline segment. The group R' in -Z₂-R'-Z₃- may be different or similar to R' in -Q₁-R'-Z₁- or -Z₄ -R'-Q₂-.

As stated, R optionally includes a hydrophilic segment and such a hydrophilic segment may very suitably be an ether segment, such as a polyether segment derivable from such polyether compounds as polyethyleneglycol, polypropyleneglycol or polybutyleneglycol. Also, a hydrophilic segment included in R may be derived from polypeptide, poly(vinyl alcohol), polyvinylpyrrolidone) or poly(hydroxyethylmethacrylate). A hydrophilic segment is preferably a polyether, *e.g.,* a poly(alkylkene glycol), such as poly(ethylene glycol), poly(propylene glycol) or poly (butylene)glycol.

In certain examples, the amorphous segment includes a hydrophilic segment. The hydrophilic segment may include polyethylene glycol in an amount of 1-80 wt%, more preferably 5-60 wt%, even more preferably 20-50 wt%, most preferably 50 wt%, based on the total weight of the hydrophilic segment. In certain examples, the phase-separated polymer is a polymer according to formula I, wherein R' is (CH₂)₄, R" is (CH₂)₄, or both R' and R" are (CH₂)₄. For example Z_{I}-Z₄ may be a urethane.

It should be appreciated that the foams described herein are comprised of a plurality of polymer chains, with each of the polymer chains comprising the phase-separated polymer, *e.g.,* a polyurethane. In many examples, the foams are substantially free of any covalent cross-linking between polymer chains included in the foam. In the context of this disclosure, the term "substantially free of any covalent cross-linking" means that one polymer chain has less than 20, less than 10, less than 6, less than 4, or less than 2 covalent bonds to other polymer chains included in the foam. In some examples, the foam is free of any covalent cross-linking between polymer chains included in the foam. In other words, each polymer chain is not covalently cross-linked to any other polymer chain included in the foam.

The term "hydrogen bonding" as used herein, refers to a partially electrostatic attraction between a hydrogen (H) atom which is bound to a more electronegative atom or group, such as nitrogen (N), oxygen (O), or fluorine (F) - the hydrogen bond donor - and another adjacent atom bearing a lone pair of electrons - the hydrogen bond acceptor. In polyurethanes, hydrogen bonding between carbonyl and N-H groups is one of the major driving forces for phase separation. Hydrogen bonds may be intermolecular (occurring between separate molecules) or intramolecular (occurring among parts of the same molecule).

In such examples, the foams described herein comprise hard/crystalline and soft/amorphous segments. The hard segments are formed via hydrogen bonding between urethane segments of each polymer chain. While not wishing to be bound by one particular theory, it is believed that urethane segments of each polymer chain are particularly susceptible to hydrogen bonding with other urethane segments in adjacent polymer chains. Accordingly, during formation of the foam, the urethane segments of each polymer chain are hydrogen bonded to, and thereby aligned with, the urethane segments of other polymer chains included in the foam. Because the urethane segments of each polymer chain are aligned with urethane segments of the other polymer chains, the polyetherester segments of each polymer chain are necessarily aligned with the polyetherester segments of other polymer chains included in the foam. The alignment of these polyetherester segments forms the soft segments of the foam. As such, because of the hydrogen bonding between urethane segments of each polymer chain, the foam exhibits a highly organized three-dimensional network structure of hard and soft segments.

The polyurethane foam of the example includes crystalline segments formed via hydrogen bonding. Further, it is believed that the crystalline segments comprising the reaction product of 1,4 butanediol and 1,4 diisocyanatobutane and the amorphous segments comprising poly(ethylene glycol) form crystalline segments and the amorphous segments that "stack" in an alternating configuration to provide a 3-dimentional porous structure which is strengthened via hydrogen bonding between the stacked crystalline segments. Further, the polyurethane foam of the example readily interacts with other polymers to hydrogen bond because it includes the crystalline segments comprising the reaction product of 1,4 butanediol and 1,4 diisocyanatobutane and the amorphous segments comprising poly(ethylene glycol). As such, the flexible film layer 40 may be selected from a polymer such as polyurethane or silicone such that the flexible film layer 40 and the flexible foam layer 38 are bonded to one another via hydrogen bonding and substantially free of covalent bonds therebetween. In this example, hydrogen bonding between the phase-separated polymer comprising crystalline segments comprising the reaction product of 1,4 butanediol and 1,4 diisocyanatobutane and amorphous segments comprising poly(ethylene glycol), and the flexible film layer 40 including silanol groups and/or urethane group occurs readily. Of course, hydrogen bonding between the flexible foam and film layers 38, 40 eliminate the need for an adhesive therebetween. As such, in many examples, the bond interface 24 between the flexible foam and film layers 38, 40 is free of an adhesive.

In one example, the removal of the flexible film layer 40 from the flexible foam layer 38 results in cohesive failure of the flexible foam layer 38 at the bond interface 24. The failure mode exhibited when the flexible film layer 40 is removed from the flexible foam layer 38 may be classified as adhesive failure, where failure occurs at the bond interface 42 between the flexible film and foam layers 38, 40, and cohesive failure, where the failure occurs within the flexible foam layer 38. As such, cohesive failure may be further described as a % area of the surface of the flexible film layer 40 which retains phase-separated polymer (foam) from the flexible foam layer 38 bonded thereto when the flexible film layer 40 is peeled from the flexible foam layer 38. As such, in some examples, the cohesive failure between the flexible film layer 40 and the flexible foam layer 38 is greater than 50, 60, 70, 80, 90, or 95%. Alternatively, the cohesive failure is described as from 50 to 99%, from 50 to 96%, from 60 to 96%, from 70 to 96%, from 80 to 93%, from 80 to 90%, from 80 to 87%, from 80 to 84%, from 83 to 99%, from 85 to 99%, from 89 to 99%, from 92 to 99%, from 95 to 99%, from 83 to 96%, from 86 to 93%, from 92-98%, from 95-98%, or 90%. Removal of the flexible film layer 40 from the flexible foam layer 38 may be accomplished manually (via hand peeling the flexible film layer 40 off the flexible foam layer 38) or in accordance with standardized test methods such as ASTM D3330 or ASTM D903.

An active agent may be dispersed within the flexible foam layer 38, and more particularly the phase-separated polymer. Typically, the active agent is a drug (*i.e.,* any pharmaceutically active compound), an antibiotics, an anti-inflammatory agent, a, corticosteroid, a hemostatic agent, an anti-allergen, an anti-cholinergic agent, an antihistamine, an anti-infective, an anti-platelet, an anti-coagulant, an anti-thrombic agent, an anti-scarring agent, an anti-proliferative agent, a chemotherapeutic agent, an anti-neoplastic agent, a pro-healing agent, decongestant, a vitamin, a hyperosmolar agent, an immunomodulator, an immunosuppressive agent, or combinations thereof.

The active agent may be located in the cell walls of the foamed phase-separated polymer. Alternatively, the active agent may be located within the voids of the foamed phase-separated polymer. When drugs are located within the cell walls of the pores, the porosity of the particular drug containing foamed portion influences the release rate of the active agent. The higher the porosity, the higher the rate of release and vice versa. Without wishing to be bound by theory, it is believed that an increased porosity results in an increased degradation rate of the phase-separated polymer and thereby an increased release rate. In other words, the degradation of the phase-separated polymer controls the release of the active agent.

The rate of release of the active agent from the phase-separated polymer may be expressed as the time required to release a certain amount of drug in a certain amount of time. Typically, 8 hours to 1.5 days are required to release 50% of the active agent from the foamed shell portion 14. In particular examples, it may be preferred that 50% of the active agent is released in a longer time, *e.g.,* in one to five days. To release about 100% (*e.g.,* more than 95%) of the active agent, a time of 4 to 14 days is generally preferred.

In one example, the active agent includes a molecule including at least one hydrogen atom, which is bound to a nitrogen, oxygen, or fluorine atom. This structure facilitates hydrogen bonding between the active agent and phase-separated polymer, *e.g.,* the polyurethane foam comprising the crystalline segments comprising the reaction product of 1,4 butanediol and 1,4 diisocyanatobutane and the amorphous segments comprising poly(ethylene glycol). In other words, the active agent may advantageously include a polymer that includes hydrogen atoms that are available to form a hydrogen bond with the crystalline segments of the first and/or second polyurethane foam. Hydrogen bonding between the active agent and the phase-separated polymer helps control and slow down the release of the active agent.

In one example, the active agent is a steroidal anti-inflammatory agent. It has been found that the relatively slow release of the active agent from the phase-separated polymer is particularly suitable for steroidal anti-inflammatory agents, such as corticosteroids. In another example, the active agent is a hemostatic agent. Of course, the closure device 10 may include both an anti-inflammatory agent, *e.g.,* a steroid, and a hemostatic agent. In various examples, the hemostatic agent includes at least one hydrogen atom bonded to a nitrogen atom, and/or at least one hydrogen atom bonded to an oxygen atom, with the hydrogen atoms being available to form a hydrogen bond with the crystalline segments of the first and/or second polyurethane foam. In such examples, molecules of the hemostatic agent and molecules of the phase-separated polymer are bonded to one another via hydrogen bonding and substantially free of covalent bonds therebetween.

In certain examples, the hemostatic agent is a chitosan hemostatic agent. The term "chitosan hemostatic agent" as used herein refers to chitosan or a salt or derivative thereof. Good results have been obtained using chitosan or chitosan acetate. Chitosan is a polysaccharide comprising D-glucosamine units (deacetylated units) and N-acetyl-D-glucosamine units (acetylated units). Chitosan may be prepared from chitin by deacetylating at least part of the N-acetyl-D-glucosamine in chitin (poly-N-acetyl-D-glucosamine) by hydrolysis. The ratio of D-glucosamine units and N-acetyl-D-glucosamine units in chitosan is typically expressed as the degree of deacetylation. The degree of deacetylation is defined as the percentage of glucosamine units in chitosan that are not acetylated. This percentage thus corresponds to the molar percentage of deacetylated units present in chitosan.

Without being bound by theory, it is believed that a higher degree of deacetylation improves the hemostatic properties. The chitosan may have a degree of deacetylation of 1-100 mol %, 25-100 mol %, 50-100 mol %, 75-100 mol %, 85-100 mol %, 90-100 mol %, 5-50 mol %, 10-35 mol %, or 10-25 mol %. The above values also apply to chitosan present in chitosan salts, as well as to chitosan derivatives (which have acetylated and deacetylated units just like chitosan itself). In additional non-limiting examples, all values and ranges of values within and including the aforementioned range endpoints are hereby expressly contemplated. Without being bound by theory, it is believed that a higher degree of deacetylation improves the hemostatic properties of the chitosan.

Suitable chitosan salts are those with the chitosan ion having a net positive charge. Accordingly, suitable chitosan salts may be salts consisting of a chitosan cation and a counter anion. For example, the chitosan hemostatic agent may be a salt of chitosan with an organic acid, in particular with a carboxylic acid such as succinic acid, lactic acid or glutamic acid. Chitosan salts may for example be selected from the group consisting of nitrate, phosphate, glutamate, lactate, citrate, acetate and hydrochloride salts of chitosan. In general, a chitosan derivative is a chitosan molecule wherein one or more of the hydroxyl groups and/or the amine group present in chitosan has been substituted. For example, the one or more hydroxyl groups may be substituted to obtain an ether or ester. The amine group may be substituted to obtain an amino group, although this generally results in a decrease in hemostatic activity. Therefore, the amine groups of chitosan are typically unsubstituted. The chitosan hemostatic agent may include or be derived from chitosan originating from animals, plants or shellfish. These sources give similar good results with respect to the hemostatic effects described above. Furthermore, synthetic chitosan may also be used. Further examples of suitable chitosan salts are chitosan esters of glutamate, succinate, phthalate or lactate, chitosan derivatives comprising one or more carboxymethyl cellulose groups, carboxymethyl chitosan. Other suitable examples of chitosan derivates are chitosan with quaternary groups (like N-trimethylene chloride, N-trimethylene ammonium). In addition, bioactive excipients such as calcitonin or 5-methylpyrrolidinone may be used. The chitosan hemostatic agent may have a molecular weight in the range of about 1-1000 kDa, 1-500 kDa, 1-250 kDa, 1-100 kDa, 10-1000 kDa, 10-500 kDa, 10-250 kDa, 10-100 kDa, 30-80 kDa, 50-1000 kDa, 50-500 kDa, 50-350 kDa, 50-250 kDa, 100-1000 kDa, 100-750 kDa, 100-500 kDa, 100-250 kDa, 150-500 kDa, 200-1000 kDa, 200-750 kDa, 200-500 kDa, 225-275 kDa, 200-300 kDa, 210-390 kDa, 90-1000 kDa,190-1000 kDa,290-1000 kDa, or 390-1000 kDa. IN additional non-limiting examples, all values and ranges of values within and including the aforementioned range endpoints are hereby expressly contemplated.

In certain examples, when the active agent is the hemostatic agent, the foamed portion including the hemostatic agent (*i.e.,* the foamed shell portion and/or the foamed core portion) has a porosity of 85-99% and a foam density of 0.03-0.07 g/cm³. Such values for the porosity and density contribute to the enhanced hemostatic activity and also provide the foam with good liquid (*e.g.,* water or blood) absorbing properties. Alternatively, the foamed portion has a foam density of 0.03-0.07 g/cm³ and a porosity of 92-98% or 95-98%. The amount of hemostatic agent may be at least 0.1 wt. %, preferably at least 2 wt. %, more preferably at least 5. wt. % of the total weight of the foam portion comprising the hemostatic agent. Notably, even this relatively small amount of hemostatic agent is sufficient to provide the foam nasal dressing with desirable hemostatic properties. Furthermore, the amount of hemostatic agent is generally less than 99 wt. %, less than 50 wt. %, or less than 35 wt. % of the total weight of the foam portion. Since the hemostatic activity of the foam nasal dressing is almost independent on hemostatic agent, high concentrations are generally neither required nor preferred. The hemostatic agent is preferably present in the foam in the form of particles, in particular polymeric particles. Examples of suitable particles are amorphous, crystalline and gel-like particles. The hemostatic agents may also be liquid, in particular when highly viscous. In case of hemostatic particles, the particles may have a size from 1-1000 µm. Preferably, particles are smaller than 150 µm. In particular good results have been obtained using particles of 5-90 µm. Small particles have a number of advantages. First, the structure of the foam is less influenced by the presence of small particles than large particles. Second, small hemostatic particles have a smaller tendency to aggregate than large particles. Furthermore, a good dispersion may be obtained using small particles. Lastly, small particles do not settle down when preparing the foam, such that a homogeneous distribution within the foam may be achieved if desirable. The hemostatic particles may be any suitable shape, but are preferably roughly spherical. The particles are preferably solid. Suitable solid particles to be used are generally insoluble and hydrophilic.

Alternatively, one or more portions of the flexible foam layer 38 may be free of the active agent. The various portions/films of the flexible foam layer 38 may each include the active agent or drug, be substantially free of the drug, or free of the drug. The term "substantially free" as used with reference to any of the active agents or drugs described herein may be defined as less than 5, 4, 3, 2, 1, 0.5, 0.1, 0.05, or 0.01 wt. %, based on a total weight of a particular portion or on a total weight of the flexible foam layer 38. The disclosure that contemplates "substantially free of" also encompasses "free of". As such, when the portions and/or device 10 are described as "substantially free of" something, *e.g.,* a drug, this descriptive language can be narrowed to "free of".

The polymer of the flexible film layer 40 may include polysiloxane, or polyurethane, or be a bioresorbable polymer including silanol groups and/or urethane groups. In other variants, the flexible film layer 40 is non-biodegradable, for example, medical grade silicone elastomer films. One such silicone elastomer film suitable for the present application is sold under the tradename SILPURAN owned by Wacker Chemie AG (Munich, Germany). The term "biodegradable" as used herein, refers to the ability of a polymer to be acted upon biochemically in general by living cells, organisms, or part of these systems, including hydrolysis, and to degrade and disintegrate into chemical or biochemical products. Further, the term "bioresorbable" as used herein, refers to the ability of being completely metabolized by the human or animal body.

One particularly advantageous implementation includes the flexible foam layer 38 being bioresorbable and the flexible film layer 40 being biocompatible and non-biodegradable. The stent 32 may be placed within the patient and left for several weeks or month. The flexible film layer 40, being non-biodegradable, remains intact while the flexible foam layer 38, being bioresorbable, resorbs after a period of time. Before, during, and after the period of resorption, patency of the frontal sinus remains preserved due to the resiliency of the flexible film layer 40. Meanwhile, the active agent(s) within the flexible foam layer 38 are delivered to the frontal sinus to facilitate an optimal wound recovery environment. For example, topical administration of a corticosteroid can be achieved while mechanically maintaining patency of the frontal sinus. Once desired to remove the stent 32, the flexible film layer 40 is the component that primarily or entirely remains, resulting in a leaner structure for easy removal through the nasal passage.

As mentioned, the stent 32 is deployed from the cartridge 36. Referring now to Figures 9-14, the cartridge 36 includes a sheath 76 having at least one sidewall 78. The sheath 76 may be tubular in form such that the sidewall 78 defines a lumen 80. The sidewall 78 includes a distal end 82 opposite a proximal end 84 with the lumen 80 extending between the distal and proximal ends 82, 84. The lumen 80 may define a volume sized to receive substantially of an entirety of the furled stent 32, more particularly between the distal and proximal ends 82, 84. The sheath 76 may be flexible so as to accommodate some contouring of the anatomy of the frontal sinus as the sheath is inserted into the same with lessened likelihood of injury.

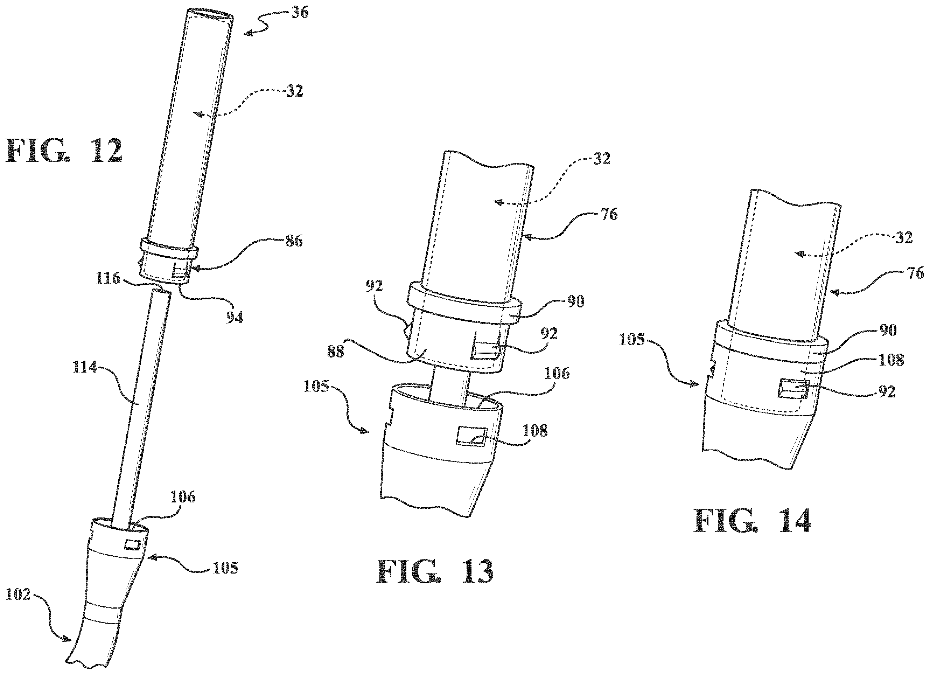

The cartridge 36 includes a cap portion 86 coupled to the sheath 76. Figures 9-14 show the cap portion 86 coupled to the proximal end 84 of the sheath 76. The cap portion 86 includes a cap body 88, a lip 90, and coupling features 92. The cap body 88 defines an aperture 94 in communication with the lumen 80 of the sheath 76. The aperture 94 of the cap body 88 may be coaxial with the lumen 80 of the sheath 76, and the respective diameters of the lumen 80 of the sheath 76 and the aperture 94 of the cap body 88 may be substantially equal. The lip 90 extends outwardly from the cap body 88. The lip 90 of Figures 9-14 is annularly disposed about the cap body 88 having a cylindrical profile. The coupling features 92 are disposed on the cap body 88. As best shown in Figure 13, the coupling features 92 may include deflectable fingers circumferentially spaced about the cap body 88 having the cylindrical profile. In a manner to be further explained, the aperture 94, the lip 90, and the coupling features 92 cooperate to removably couple the cartridge 36 to the applicator device 34. The coupling features may be implemented in alternative ways, such as notches, grooves, threads, and the like. The cartridge 36 may be a disposable component of the system 30.

The cartridge 36 is configured to removably receive the stent 32 prior to or after being coupled with the applicator device 34. In one example, the stent 32 is preloaded within the sheath 76 of the cartridge 36. Figures 9-11 show certain steps of loading the stent 32 (the variant of the stent 32*b* shown in Figure 4B) into the cartridge 36. The stent 32*b* is shown in an unfurled or unrestricted state. The stent 32*b* may be furled manually to assume a substantially cylindrical profile. The first body portion 60 and the second body portion 62 may be independently furled other than at the junction 64, or manipulated to be furled as a unit. When furled, the flexible foam layer 40 at least partially defines the outer annular aspect 56 of the stent 32*b* as shown, and the flexible film layer 38 at least partially defines the inner annular aspect 58 of the stent 32*b*. The furled stent 32*b* may be inserted into the cartridge 36 and directed through the lumen 80 such that the lower end 48 is directed through the aperture 94 of the cap portion 86 to be approximately in registration with a base of the cap portion 86. Figure 11 best shows the lower end 48 defining the inner annular aspect 58 at or near the base of the cap portion 86. Owing to the resiliency of the flexible film layer 40, it is appreciated that stent 32b may unfurl slightly within the lumen 80 subject to the constraints provided by the sheath 76. The interior passageway 57 may become slightly more patent with the slight unfurling within the sheath 76. The furled stent 32 within the cartridge 36 may be considered a cartridge assembly. It should be appreciated that various types of stents may be utilized in the cartridge, and the systems and methods described herein could be used to place the stents, and/or cartridges in locations other than the frontal sinus, including but not limited to, other sinus spaces, intravenously, in arthroscopic procedures, in nerve remediation, in joints, and in various ear and throat applications.

Referring now to Figures 1-3 and Figures 12-14, the applicator device 34 includes a housing 96 defining a bore 100. The housing 96 may include a distal housing portion 102 coupled to a proximal housing portion 104 with the bore 100 extending through at least a portion of each of the distal and proximal housing portions 102, 104. The housing 96 is configured to be removably coupled with the cartridge 36. In particular, the distal housing portion 102 may include a head 105 near a distal end of the distal housing portion 102. The head 105 defines an opening 106 in communication with the bore 100. Near the opening 106, the head 105 includes coupling features 108 complementary to the coupling features 92 of the cap portion 86 of the cartridge 36. Figure 13 best shows the coupling features 108 as keyways circumferentially disposed about the head 105 in positioned complementary to the circumferential positions of the keys comprising the coupling features 92. Further, the opening 106 is sized to slidably and snugly receive the cap body 88 of the cap portion 86. With particular reference to Figures 13 and 14, the cap body 88 is directed through the opening 106 until the lip 90 abuts the distal end of the applicator device 34. Simultaneously or subsequently, the rotational orientation of the cartridge 36 relative to the applicator device 34 is arranged such that the complementary coupling features 92, 108 are in registration and engage, thereby selectively coupling the cartridge 36 and the applicator device 34.

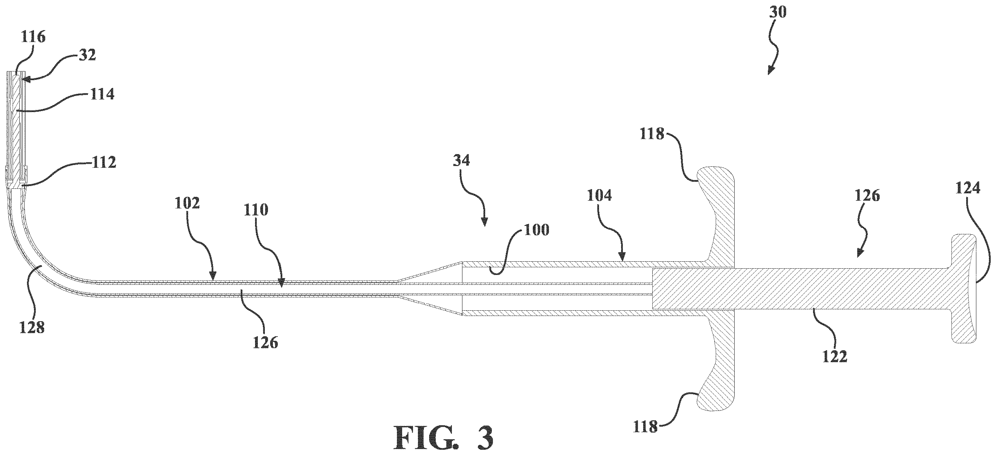

The furled stent 32 may be inserted into the sheath 76 of the cartridge 36 prior to removably coupling the cap portion 86 to the applicator device 34. The applicator device 34 includes a drive member 110 and a plunger element 112 coupled to the drive member 110. The drive member 110 may include a mandrel 114 extending distal to the plunger element 112. As appreciated from Figures 3, 12 and 13, the mandrel 114 extends through the passageway 57 defined by the inner annular aspect 58 of the stent 32 when the stent 32 is disposed within the cartridge 36 and the cartridge 36 coupled to the applicator device 34. In particular, the aperture 94 of the cap portion 86 and the lumen 80 of the sheath 76 are sized to receive the mandrel 114 of the applicator device 34. Further, the interior passageway 57 may become slightly more patent with the slight unfurling within the sheath 76, as mentioned. Thus, the user aligns a distal tip 116 of the mandrel 114 with the interior passageway 57 furled within the cartridge 36. The user directs the mandrel 114 through the interior passageway 57 until the lip 90 abuts the distal end of the applicator device 34 and the complementary coupling features 92, 108 are engaged, as previously described. The mandrel 114 may provide control over positioning of the stent 32 within the frontal sinus during deployment from the cartridge 36.

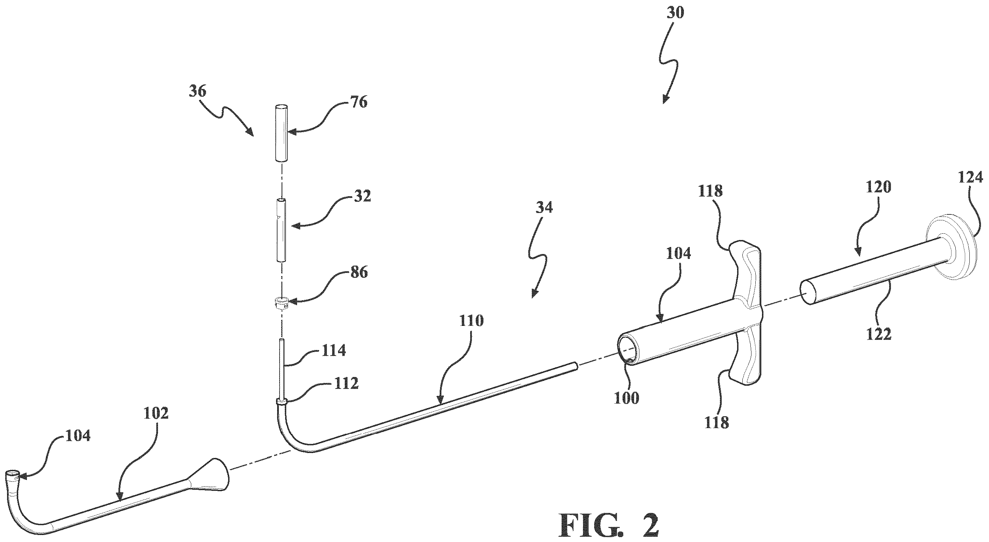

Figures 1-3 show the housing 96 configured to be grasped by the user, for example with a single hand. In particular, the housing 96 may include a handle 118 extending laterally outward from the proximal housing portion 104 with the handle 118 contoured to be handled by fingers of the single hand of the user. The applicator device 34 further includes an actuator 120 coupled to the drive member 110, and more particularly a proximal end of the drive member 110 extending through the bore 100. With continued reference to Figures 2 and 3, the actuator 120 may include a push rod 122 snugly and slidably disposed within the bore 100 of the proximal housing portion 104, and a control surface 124 coupled to the push rod 122. The control surface 124 is configured to receive an input from the user to move the push rod 122 relative to the housing 96. In particular, the control surface 124 is sized and positioned to be maintained in its position as the user provides the input to the handle 118 to retract the proximal housing portion 104 relative to the actuator 120. Additionally or alternatively, the user may advance the push rod 122 while maintaining the position of the proximal housing portion 104. The relative movement of the push rod 122 relative to the housing 96 urges movement of the drive member 110 relative to the housing 96.

The push rod 122 abuts the proximal end of the drive member 110, as mentioned. The drive member 110 may include a proximal portion 126 and a distal portion 128 positioned distal to the proximal portion 126. The proximal and distal portions 126, 128 of the drive member 110 may be discrete components coupled to one another, or of unitary construction. The proximal portion 126 may be substantially rigid, whereas the distal portion 128 may be flexible relative to the proximal portion 126. For example, the distal portion 128 (and at least an overlying corresponding length of the distal housing portion 102) may be malleable to permit the user to apply a force to impart a desired curve to the applicator device 34. Additionally or alternatively, the distal portion 128 of the drive member 110 may be malleable and the overlying corresponding length of the distal housing portion 102 may be flexible to assume the desired curve imparted to the applicator device 34. Among other advantages, imparting the desired curve to the applicator device 34 may permit the user to access more remote anatomy of the frontal sinus.

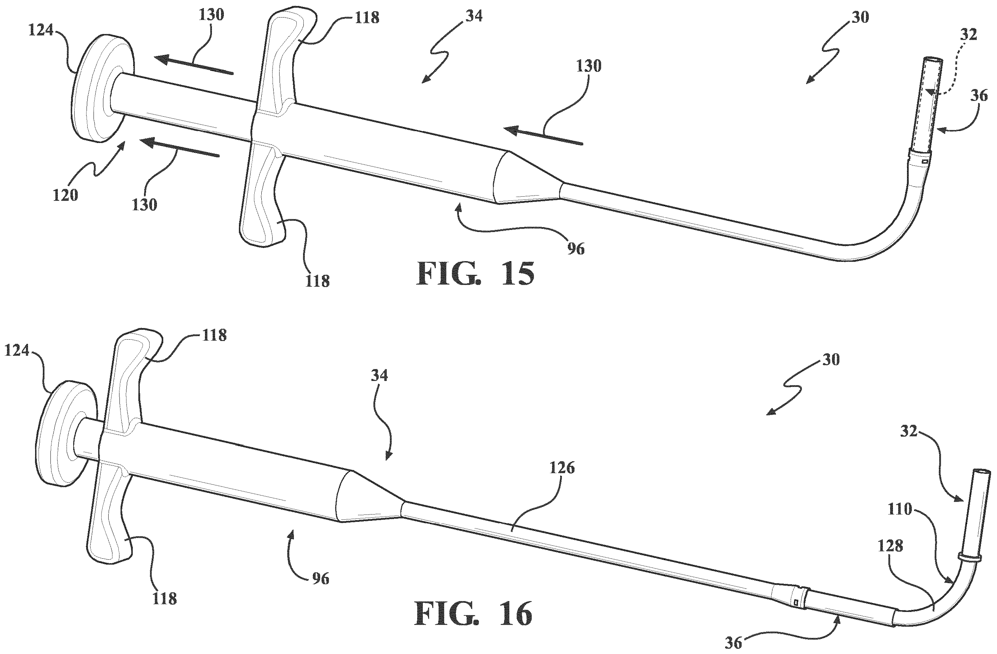

Figure 3 shows the plunger element 112 as an annular flange extending from the drive member 110. The mandrel 114 may extend distally from the plunger element 112. The aperture 94 of the cap portion 86 of the cartridge 36 is sized to receive the plunger element 112 when the cap portion 86 is coupled to the housing 96 of the applicator device 34. More particularly, an inner diameter of the aperture 94 is greater than an outer diameter of the plunger element 112 such that the plunger element 112 may pass through the aperture 94 with actuation of the applicator device 34. Likewise, the lumen 80 of the sheath 76 of the cartridge 36 is greater than the outer diameter of the plunger element 112 such that the plunger element 112 may pass through the lumen 80 with actuation of the applicator device 34 when the stent 32 is deployed from the sheath 76. With further reference to Figures 15 and 16, Figures 3 and 15 shows the cartridge 36 coupled to the applicator device 34 with the furled stent 32 disposed within the cartridge 36. The mandrel 114 extends through the interior passageway 57 of the furled stent 32 with the distal tip 116 of the mandrel 114 near the distal end 82 of the sheath 76. The distal housing portion 102 and the distal portion 128 of the drive member 110 are in a curved configuration. The user grasp the applicator device 34, for example, with a single hand with fingers on the handle 118 and the thumb on the control surface 124 of the actuator 120. When ready to deploy the stent 32, an input (*e.g.,* squeezing motion) is provided to one or both of the handle 118 and the control surface 124 to move the housing 96 relative to the actuator 120 in the direction of arrows 130 of Figure 15. As shown in Figure 16, the housing 96 moves toward the control surface 124, and more particularly the distal housing portion 102 is retracted proximally relative to the drive member 110. Owing to the flexible nature of the distal housing portion 102 and the malleable nature of the distal portion 128 of the drive member 110, the distal housing portion 102 straightens during retraction to conform to the shape of the proximal portion 126 of the drive member 110.

The cartridge 36 and the distal housing portion 102 are coupled to one another with the complementary coupling features 92, 108, and the plunger element 112 supports the lower end 48 of the stent 32. Further, as mentioned, the aperture 94 of the cap portion 86 is sized to permit passage of the plunger element 112. As such, the retraction of the distal housing portion 102 results in retraction of the cartridge 36 over the drive member 110, as shown in Figure 16, and the flexible nature of the sheath 76 of the cartridge 36 permits the cartridge 36 to accommodate the imparted curve of the drive member 110. With the stent 32 is constrained from moving in a corresponding manner by the plunger element 112, the stent 32 is exposed beyond the cartridge 36, thereby deploying the stent 32. The stent 32, in an unrestricted state, unfurls in manners previously described.

An exemplary method of using the system 30 will be described with reference to the anatomy of Figure 17. The frontal sinus (FS) includes the frontal sinus cavities (FSC) opening into the nasal passage (NP) through the frontal sinus openings (FSO) (*i.e.,* ostia). The nasal passage (NP) is at least partially defined by the superior turbinate (ST), middle turbinate (MT), and inferior turbinate (IT) for warming, humidifying, and filtering the air. The nasal sinus also includes the maxillary sinus cavity (MSC) and ethmoid sinus (ES), among others. The frontal sinus cavity, nasal passage, maxillary sinus cavity, and the like, are defined by the mucosal wall (MW). Of particular interest if the mucosal wall defining the frontal sinus opening. The system 30 is provided including the stent 32, the applicator device 34, and the cartridge 36. The stent 32 may be preloaded in the cartridge 36 (*e.g.,* hermetically sealed with a film cover). Alternatively, the stent 32 may be altered to a desired profile, for example, by cutting the cutouts 68 of the stent 32 to a desired shape. The stent 32 is manually furled and loaded into the sheath 76 of the cartridge 36. The flexible film layer 40 at least partially defines the inner annular aspect 58 of the stent 32, and the flexible foam layer 38 at least partially defines the outer annular aspect 56 of the stent 32. The distal end 82 of the sheath 76 is directed into and through the nasal passage, as shown in Figure 17. The distal end 82 of the sheath 76 may be directed through the frontal sinus opening and positioned slightly superior the frontal sinus opening. The input is provided to the applicator device 34 to retract the cartridge 36 relative to the drive member 110 to deploy the stent 32. The first body portion 60 of the stent 32 may be positioned in the frontal sinus cavity, and the second body portion 62 may be positioned in the frontal sinus opening and/or the nasal passage. Owing to the front and second body portions 60, 62 being able to independently unfurl, the first body portion 60 unfurls to a relatively greater extent to create an interference with the anatomy defining the frontal sinus cavity near the frontal sinus opening. The interference facilitates retention of the stent 32 within the frontal sinus. The second body portion 62 unfurls to create and maintain patency of the frontal sinus opening. Further, the flexible foam layer 38 in direct contact with the mucosal wall may deliver the active agent(s). The applicator device 34 is removed from the frontal sinus, and the cartridge 36 decoupled from the applicator device 34 and disposed of in a suitable manner. It is also contemplated that the stent may be pre-loaded within the cartridge, with both the stent and cartridge packaged together in sterile state.

It is readily appreciated that the system 30 is well suited to deploy the stent 32 for maintaining patency of the frontal sinus opening. It is contemplated that the system 30 may be used to deploy the stent 32 within any orifice or passage of the sinus, including those in communication with the ethmoidal sinus, sphenoidal sinus, maxillary sinus, passages defined by the superior, middle, and/or inferior turbinates. It is further contemplated that the system 30 may be used other passages of the that the system 30 may be used to deploy the stent 32 other orifices and passages of the nose, ear, throat, brain, and the like.

## Claims

1. A system (30) for stenting a frontal sinus cavity, the system comprising:
an applicator device (34) comprising a housing (96) defining a bore (100) and adapted to be grasped with a single hand of a user, an actuator (120), and a drive member (110) coupled to the actuator and with the drive member configured to be moved within the bore in response to one or both of the housing and the actuator receiving an input from the user; a disposable cartridge removably coupled to the housing and comprising a tubular sheath (76) defining a lumen (80), and a cap portion (86) coupled to the tubular sheath and removably coupled to the housing of the applicator device; and
a stent (32) comprising:
a flexible foam layer (38) having a porosity of greater than 80%, the flexible foam layer comprising a polyurethane including amorphous segments, and crystalline segments formed via hydrogen bonding;
a flexible film layer (40) arranged in a stacked configuration and comprising a polymer which is hydrogen bonded to the crystalline segments of the flexible foam layer at a bond interface; and
an active agent within the flexible foam layer;
wherein the stent is configured to be furled and inserted within the tubular sheath, the flexible film layer having a resilience sufficient to unfurl the stent when positioned in an unrestricted state in the frontal sinus cavity.

2. The system as set forth in claim 1, wherein the bond interface is free of adhesive, and the flexible film layer (40) and the flexible foam layer (38) are bonded directly to one another via hydrogen bonding.

3. The system as set forth in claim 1 or 2, wherein the stent (32) assumes a cylindrical profile when furled, with the flexible foam layer (38) at least partially defining an outer annular aspect (56) of the stent and the flexible film layer (40) at least partially defining an inner annular aspect (58) of the stent.

4. The system as set forth in any one of claims 1-3, wherein the stent (32) further comprises a first body portion (60) and a second body portion (62) coupled at a junction (64) extending partially between opposing sides of the stent such that the first and second body portions are configured to be independently furled and/or unfurled other than at the junction.

5. The system as set forth in any one of claims 1-4, wherein the tubular sheath (76) comprises at least one sidewall (78) defining a proximal end (84) and a distal end (82) opposite the proximal end with lumen (80) extending between the proximal and distal ends and defining a sheath volume, wherein the furled stent (32) comprises a length defined between opposing ends being less than a length of the tubular sheath such that the furled stent is entirely disposed within the sheath volume prior to deploying the stent from the sheath volume.

6. The system as set forth in any one of claims 1-5, wherein the inner annular aspect (58) of the furled stent (32) defines an interior passageway (57), wherein the drive member (110) comprises a distal end (82) opposite a proximal end (84) coupled to the actuator (120), and a mandrel (114) extending from the distal end and through the interior passageway of the stent when the stent is disposed within the flexible sheath.

7. The system as set forth in claim 6, wherein the cap portion (86) defines an aperture (94) in communication with the lumen (80) of the tubular sheath (76) with the mandrel (114) of the drive member (110) extending through the aperture of the cap portion when the cap portion is coupled to the housing (96).

8. The system as set forth in claim 7, wherein the drive member (110) further comprises a plunger element (112) with the mandrel (114) extending from the plunger element, wherein the aperture (94) of the cap portion (86) is sized to receive the plunger element of the applicator device (34) when the cap portion is coupled to the housing (96).

9. The system as set forth in claim 7, wherein the aperture (94) is sized to movably receive the drive member (110) of the applicator device (34) such that the drive member extends through the aperture and the lumen (80) of the tubular sheath (76) when the stent (32) is deployed from the disposable cartridge.

10. The system as set forth in any one of claims 1-9, wherein the housing (96) and the cap portion (86) further comprise complementary coupling features (92, 108) configured to removably couple the disposable cartridge with the applicator device (34).

11. The system as set forth in any one of claims 1-10, wherein at least a portion of the housing (96) is flexible and at least a portion of the drive member (110) is malleable and configured to receive another input from the user imparting a desired curve of the disposable cartridge, and to maintain the desired curve of the disposable cartridge relative to the housing upon release of the another input.

12. The system as set forth in any one of claims 1-11, wherein the actuator (120) comprises a push rod (122) movably disposed within the bore of the housing (96).

13. The system as set forth in any one of claims 1-12, wherein the flexible film layer (40) comprises polysiloxane or polyurethane or wherein the flexible film layer comprises polymer comprising silanol groups and/or urethane groups.

14. The system as set forth in any one of claims 1-13, wherein the flexible foam layer (38) is bioresorbable, and/or wherein the active agent is selected from corticosteroids, hemostatic agents, and combinations thereof.

## Patentansprüche

1. System (30) zum Einsetzen eines Stents in eine Stirnhöhle, wobei das System umfasst:
eine Applikatorvorrichtung (34), umfassend ein Gehäuse (96), das eine Bohrung (100) definiert und so ausgelegt ist, dass es mit einer Hand eines Benutzers ergriffen werden kann, einen Aktuator (120) und ein Antriebselement (110), das mit dem Aktuator gekoppelt ist und so konfiguriert ist, dass es innerhalb der Bohrung bewegt wird, wenn das Gehäuse und/oder der Aktuator eine Eingabe vom Benutzer erhalten; eine Einwegkartusche, die abnehmbar mit dem Gehäuse verbunden ist und eine röhrenförmige Hülle (76), die ein Lumen (80) definiert, sowie einen mit der röhrenförmigen Hülle verbundenen und abnehmbar mit dem Gehäuse der Applikatorvorrichtung verbundenen Kappenabschnitt (86) umfasst; und
einen Stent (32), umfassend:
eine flexible Schaumstoffschicht (38) mit einer Porosität von mehr als 80 %, wobei die flexible Schaumstoffschicht ein Polyurethan umfasst, das amorphe Segmente und durch Wasserstoffbrückenbindungen gebildete kristalline Segmente enthält;
eine flexible Filmschicht (40), die in einer gestapelten Anordnung angeordnet ist und ein Polymer umfasst, das an einer Bindungsgrenzfläche über Wasserstoffbrückenbindungen mit den kristallinen Segmenten der flexiblen Schaumstoffschicht verbunden ist; und
einen Wirkstoff innerhalb der flexiblen Schaumstoffschicht;
wobei der Stent so konfiguriert ist, dass er in der röhrenförmigen Hülle aufgerollt und in diese eingeführt werden kann, wobei die flexible Filmschicht eine Elastizität aufweist, die ausreicht, um den Stent zu entfalten, wenn sie in einem unbeschränkten Zustand in der Stirnhöhle positioniert ist.

2. System nach Anspruch 1, wobei die Bindungsgrenzfläche frei von Klebstoff ist und die flexible Filmschicht (40) und die flexible Schaumstoffschicht (38) über Wasserstoffbrückenbindungen direkt miteinander verbunden sind.

3. System nach Anspruch 1 oder 2, wobei der Stent (32) im zusammengerollten Zustand ein zylindrisches Profil annimmt, wobei die flexible Schaumstoffschicht (38) zumindest teilweise einen äußeren ringförmigen Aspekt (56) des Stents definiert und die flexible Filmschicht (40) zumindest teilweise einen inneren ringförmigen Aspekt (58) des Stents definiert.

4. System nach einem der Ansprüche 1 bis 3, wobei der Stent (32) ferner einen ersten Körperabschnitt (60) und einen zweiten Körperabschnitt (62) umfasst, die an einer Verbindung (64) miteinander verbunden sind, die sich teilweise zwischen gegenüberliegenden Seiten des Stents erstreckt, so dass der erste und der zweite Körperabschnitt so ausgebildet sind, dass sie unabhängig voneinander, mit Ausnahme an der Verbindung, aufgerollt und/oder entrollt werden können.

5. System nach einem der Ansprüche 1 bis 4, wobei die röhrenförmige Hülle (76) mindestens eine Seitenwand (78) aufweist, die ein proximales Ende (84) und ein dem proximalen Ende gegenüberliegendes distales Ende (82) definiert, wobei sich ein Lumen (80) zwischen dem proximalen und dem distalen Ende erstreckt und ein Hüllenvolumen definiert, wobei der aufgerollte Stent (32) eine Länge aufweist, die zwischen den gegenüberliegenden Enden definiert ist und kleiner ist als eine Länge der röhrenförmigen Hülle, so dass der aufgerollte Stent vor dem Entfalten des Stents aus dem Hüllenvolumen vollständig innerhalb des Hüllenvolumens angeordnet ist.

6. System nach einem der Ansprüche 1 bis 5, wobei der innere ringförmige Aspekt (58) des aufgerollten Stents (32) einen inneren Durchgang (57) definiert, wobei das Antriebselement (110) ein distales Ende (82) gegenüber einem proximalen Ende (84) aufweist, das mit dem Aktuator (120) verbunden ist, sowie einen Dorn (114), der sich vom distalen Ende aus durch den inneren Durchgang des Stents erstreckt, wenn sich der Stent innerhalb der flexiblen Hülle befindet.

7. System nach Anspruch 6, wobei der Kappenabschnitt (86) eine Öffnung (94) definiert, die mit dem Lumen (80) der röhrenförmigen Hülle (76) in Verbindung steht, wobei sich der Dorn (114) des Antriebselements (110) durch die Öffnung des Kappenabschnitts erstreckt, wenn der Kappenabschnitt mit dem Gehäuse (96) verbunden ist.

8. System nach Anspruch 7, wobei das Antriebselement (110) ferner ein Kolbenelement (112) umfasst, wobei sich der Dorn (114) von dem Kolbenelement erstreckt, und wobei die Öffnung (94) des Kappenabschnitts (86) so dimensioniert ist, dass sie das Kolbenelement der Applikatorvorrichtung (34) aufnimmt, wenn der Kappenabschnitt mit dem Gehäuse (96) verbunden ist.

9. System nach Anspruch 7, wobei die Öffnung (94) so dimensioniert ist, dass sie das Antriebselement (110) der Applikatorvorrichtung (34) beweglich aufnimmt, sodass sich das Antriebselement durch die Öffnung und das Lumen (80) der röhrenförmigen Hülle (76) erstreckt, wenn der Stent (32) aus der Einwegkartusche entfaltet wird.

10. System nach einem der Ansprüche 1 bis 9, wobei das Gehäuse (96) und der Kappenabschnitt (86) ferner komplementäre Kupplungsmerkmale (92, 108) aufweisen, die so ausgebildet sind, dass sie die Einwegkartusche lösbar mit der Applikatorvorrichtung (34) verbinden.

11. System nach einem der Ansprüche 1 bis 10, wobei zumindest ein Abschnitt des Gehäuses (96) flexibel ist und zumindest ein Abschnitt des Antriebselements (110) verformbar ist und so ausgelegt ist, dass er eine weitere Eingabe des Benutzers aufnimmt, die der Einwegkartusche eine gewünschte Krümmung verleiht, und dass er die gewünschte Krümmung der Einwegkartusche relativ zum Gehäuse nach dem Wegfallen der weiteren Eingabe aufrechterhält.

12. System nach einem der Ansprüche 1 bis 11, wobei der Aktuator (120) eine Schubstange (122) umfasst, die beweglich innerhalb der Bohrung des Gehäuses (96) angeordnet ist.

13. System nach einem der Ansprüche 1 bis 12, wobei die flexible Filmschicht (40) Polysiloxan oder Polyurethan umfasst oder wobei die flexible Filmschicht ein Polymer umfasst, das Silanolgruppen und/oder Urethangruppen enthält.

14. System nach einem der Ansprüche 1 bis 13, wobei die flexible Schaumstoffschicht (38) bioresorbierbar ist und/oder wobei der Wirkstoff ausgewählt ist aus Kortikosteroiden, Hämostatika und Kombinationen davon.

## Revendications

1. Système (30) pour poser une endoprothèse dans le sinus frontal, le système comprenant:
un dispositif applicateur (34) comprenant un boîtier (96) délimitant d'un orifice (100) et adapté pour être saisi d'une seule main par un utilisateur, un actionneur (120), et un élément d'entraînement (110) couplé à l'actionneur, l'élément d'entraînement étant conçu pour se déplacer à l'intérieur de l'orifice dès que le boîtier et/ou l'actionneur reçoit une intervention de l'utilisateur; une cartouche jetable couplée de manière amovible au boîtier et comprenant une gaine tubulaire (76) délimitant une lumière (80), et une partie formant capuchon (86) couplée à la gaine tubulaire et couplée de manière amovible au boîtier du dispositif applicateur; et
une endoprothèse (32) comprenant:
une couche de mousse souple (38) présentant une porosité supérieure à 80 %, la couche de mousse souple étant constituée d'un polyuréthane comprenant des segments amorphes et des segments cristallins formés par des liaisons hydrogène;
une couche de film souple (40) empilée et comprenant un polymère qui est lié par des liaisons hydrogène aux segments cristallins de la couche de mousse souple au niveau d'une interface de liaison; et
un agent actif présent dans la couche de mousse souple;
dans lequel l'endoprothèse est conçue pour être repliée et insérée à l'intérieur de la gaine tubulaire, la couche de film souple présentant une élasticité suffisante pour déplier l'endoprothèse lorsqu'elle est placée à l'état libre dans le sinus frontal.

2. Système selon la revendication 1, dans lequel l'interface de liaison est exempte d'adhésif, et la couche de film souple (40) et la couche de mousse souple (38) sont liées directement l'une à l'autre par des liaisons hydrogène.

3. Système selon la revendication 1 ou 2, dans lequel l'endoprothèse (32) présente un profil cylindrique lorsqu'elle est enroulée, la couche de mousse souple (38) délimitant au moins partiellement une face annulaire externe (56) de l'endoprothèse et la couche de film souple (40) délimitant au moins partiellement une face annulaire interne (58) de l'endoprothèse.

4. Système selon l'une quelconque des revendications 1 à 3, dans lequel l'endoprothèse (32) comprend en outre une première partie de corps (60) et une deuxième partie de corps (62) reliées au niveau d'une jonction (64) s'étendant partiellement entre les côtés en regard de l'endoprothèse, de telle sorte que les première et deuxième parties de corps soient conçues pour être enroulées et/ou déroulées indépendamment l'une de l'autre, à l'exception de la jonction.

5. Système selon l'une quelconque des revendications 1 à 4, dans lequel la gaine tubulaire (76) comprend au moins une paroi latérale (78) délimitant une extrémité proximale (84) et une extrémité distale (82) en regard de l'extrémité proximale, avec une lumière (80) s'étendant entre les extrémités proximale et distale et délimitant un volume de gaine, dans lequel l'endoprothèse enroulée (32) présente une longueur définie entre des extrémités en regard qui est inférieure à la longueur de la gaine tubulaire, de sorte que l'endoprothèse enroulée est entièrement logée à l'intérieur du volume de gaine avant le déploiement l'endoprothèse à partir du volume de gaine.

6. Système selon l'une quelconque des revendications 1 à 5, dans lequel la partie annulaire interne (58) l'endoprothèse enroulée (32) délimite un passage intérieur (57), dans lequel l'élément d'entraînement (110) comprend une extrémité distale (82) en regard d'une extrémité proximale (84) couplée à l'actionneur (120), et un mandrin (114) s'étendant depuis l'extrémité distale et traversant le passage intérieur de l'endoprothèse lorsque l'endoprothèse est disposée à l'intérieur de la gaine flexible.

7. Système selon la revendication 6, dans lequel la partie de capuchon (86) délimite une ouverture (94) en communication avec la lumière (80) de la gaine tubulaire (76), le mandrin (114) de l'élément d'entraînement (110) s'étendant à travers l'ouverture de la partie de capuchon lorsque la partie de capuchon est couplée au boîtier (96).

8. Système selon la revendication 7, dans lequel l'élément d'entraînement (110) comprend en outre un élément de piston (112), le mandrin (114) s'étendant à partir de l'élément de piston, l'ouverture (94) de la partie de capuchon (86) étant dimensionnée pour recevoir l'élément de piston du dispositif applicateur (34) lorsque la partie de capuchon est couplée au boîtier (96).

9. Système selon la revendication 7, dans lequel l'ouverture (94) est dimensionnée pour recevoir de manière mobile l'élément d'entraînement (110) du dispositif applicateur (34), de telle sorte que l'élément d'entraînement s'étende à travers l'ouverture et la lumière (80) de la gaine tubulaire (76) lorsque l'endoprothèse (32) est déployé à partir de la cartouche jetable.

10. Système selon l'une quelconque des revendications 1 à 9, dans lequel le boîtier (96) et la partie de capuchon (86) comportent en outre des éléments d'accouplement complémentaires (92, 108) conçus pour accoupler de manière amovible la cartouche jetable au dispositif applicateur (34).

11. Système selon l'une quelconque des revendications 1 à 10, dans lequel au moins une partie du boîtier (96) est souple et au moins une partie de l'élément d'entraînement (110) est malléable et conçue pour recevoir une autre intervention de l'utilisateur afin de conférer à la cartouche jetable une courbure souhaitée, et pour maintenir la courbure souhaitée de la cartouche jetable par rapport au boîtier dès que l'autre intervention cesse.

12. Système selon l'une quelconque des revendications 1 à 11, dans lequel l'actionneur (120) comprend une tige de poussée (122) disposée de manière mobile à l'intérieur de l'orifice du boîtier (96).

13. Système selon l'une quelconque des revendications 1 à 12, dans lequel la couche de film souple (40) est constituée de polysiloxane ou de polyuréthane, ou dans lequel la couche de film souple est constituée de polymère comportant des groupes silanol et/ou des groupes uréthane.

14. Système selon l'une quelconque des revendications 1 à 13, dans lequel la couche de mousse souple (38) est biorésorbable, et/ou dans lequel le principe actif est choisi parmi les corticostéroïdes, les agents hémostatiques et leurs combinaisons.
